# EUROPEAN PATENT APPLICATION

(11) **EP 3 750 960 A1**
(43) Date of publication of application: **16.12.2020**
(21) Application number: 18904621.2
(22) Date of filing: 08.02.2018
(51) Int. Cl.: C08L 101/00, C08K 3/34, C08L 33/14

(54) **POWDER-CONTAINING COMPOSITION, POWDER FOR AQUEOUS SOLVENT, AND PRODUCTION METHOD OF POWDER FOR AQUEOUS SOLVENT**

(71) Applicant: Shiseido Company, Ltd., Chuo-ku Tokyo 104-0061 (JP)
(72) Inventor: ANDO Yumi, Yokohama-shi, Kanagawa 224-8558 (JP)
(74) Representative: Ter Meer Steinmeister & Partner
(86) International application number: PCT/JP2018/004467
(87) International publication number: WO 2019/155584

(57) **Abstract**

A powder-containing composition contains an aqueous solvent, a cationic polymer that is present in the aqueous solvent and a powder that bears a negative charge on a surface in the aqueous solvent and has a BET specific surface area of less than 50 m²/g. A ratio of the mass in the composition of the cationic polymer to a total surface area in the composition of the powder is 1 µg/m² to 100,000 µg/m².

## Description

### Technical Field

The present disclosure relates to a composition comprising a powder. The present disclosure relates to a powder applicable to an aqueous solvent. The present disclosure relates to a method for manufacturing the powder.

### Background Art

Products in which a powder is added to an aqueous solvent have been used in a wide field such as foods, paints, cosmetics, inks, ceramics, and the like. Technique for enhancing dispersibility of powders has been developed for such products (refer to Patent Literature 1, for example).

A powder-containing cosmetic described in Patent Literature 1 comprises at least silicic anhydride, a cationic polymer, a nonionic surfactant, and water, wherein: the cationic polymer is one or more selected from cationic cellulose, a polymer of dimethyl diallyl ammonium chloride, dimethyl diallyl ammonium chloride/acrylamide copolymer, acrylic acid/dimethyl diallyl ammonium chloride copolymer, and acrylic acid/dimethyl diallyl ammonium chloride/acrylamide copolymer; the content of the cationic polymer is 0.01% by mass to 3% by mass; the content of the nonionic surfactant is 0.05% by mass to 5% by mass; the mass ratio between the cationic polymer and the nonionic surfactant (cationic polymer/nonionic surfactant) is 0.02 or greater and 10 or less; pH at 25°C is pH 3 or greater and less than pH 7; and the cosmetic is stored in a non-aerosol spray container to be used.

### Citation List

### Patent Literature

Patent Literature 1: Japanese Patent No. 5832118 B.

### Summary of Invention

### Technical Problem

In a liquid cosmetic comprising a powder, for example, the powder sediments during storage. In this case, if the sediment powder aggregates or solidifies at the bottom of the container, the user cannot use the powder component. Thus, even if the powder sediments, it is desired that aggregation of the powder is suppressed, and a user can easily redisperse the powder by a simple action such as shaking the container, or the like.

In other cases where a cosmetic sheet or a cleansing sheet in which a sheet such as a non-woven fabric is impregnated with a liquid cosmetic or a liquid cleansing agent including a powder is prepared, a liquid component including the powder is prepared in advance, and then the sheet is impregnated with the liquid component. Upon impregnating the sheet with the liquid component, the powder needs to be dispersed in the liquid component. However, if the powder aggregates in a tank where the liquid component is prepared, a process of disintegrating the aggregated powder becomes necessary before impregnating the sheet with the liquid component. Therefore, in such case, it is desired that aggregation of the powder is suppressed, and the powder can be easily redispersed by rotating the tank, and the like.

In particular, a composition having an enhanced redispersibility compared to the powder-containing cosmetic described in Patent Literature 1 is demanded.

Furthermore, a powder capable of achieving redispersibility as described above can enhance convenience and expand forms of use.

### Solution to Problem

According to a first aspect of the present disclosure, a powder-containing composition including an aqueous solvent, a cationic polymer that is present in the aqueous solvent, and a powder that bears a negative charge on a surface in the aqueous solvent and has a BET specific surface area of less than 50 m²/g is provided. A ratio of a mass in the composition of the cationic polymer to a total surface area in the composition of the powder is 1 µg/m² to 100,000 µg/m².

According to a second aspect of the present disclosure, a powder for an aqueous solvent including a particle bearing a negative charge on a surface in the aqueous solvent and having a BET specific surface area of less than 50 m²/g, and a cationic polymer adhered to the surface of the particle is provided. A ratio of a mass of the cationic polymer to a total surface area of the particle is 1 µg/m² to 100,000 µg/m².

According to a third aspect of the present disclosure, a method for manufacturing a powder for an aqueous solvent is provided, the method including mixing, in the aqueous solvent, a powder that bears a negative charge on a surface in an aqueous solvent and has a BET specific surface area of less than 50 m²/g, and 1 µg/m² to 100,000 µg/m² of a cationic polymer relative to a total surface area of the powder, and isolating the mixture prepared in the mixing step from the aqueous solvent.

### Effect of Invention

In the powder-containing composition of the present disclosure, even if the power sediments, aggregation and solidification of the powder are suppressed. In the powder-containing composition of the present disclosure, even if the power sediments, the powder can be easily redispersed by allowing the solvent to move.

In the powder for the aqueous solvent of the present disclosure, even if the powder sediments in the aqueous solvent, aggregation and solidification are suppressed. The powder for the aqueous solvent can be easily redispersed by allowing the solvent to move even if the powder sediments in the aqueous solvent. According to the powder for the aqueous solvent, the powder-containing composition of the present disclosure can be manufactured easily.

According to the method, of the present disclosure, for manufacturing the powder for the aqueous solvent, the powder for the aqueous solvent of the present disclosure can be manufactured easily.

### Advantageous Effects of Invention

Preferred modes of each aspect mentioned above are described in the following.

According to a prefered mode of the above first aspect, the cationic polymer comprises, relative to a molecular weight of the cationic polymer, 15% to 85% of a first component having a structure represented by undermentioned Chem. 1, and 15% to 85% of a second component having a structure represented by undermentioned Chem. 2 or Chem. 3.

According to a prefered mode of the above first aspect, the cationic polymer is adsorbed to the powder.

According to a prefered mode of the above first aspect, the powder has an aspect ratio of two or more, the aspect ratio being an average value of a ratio of a second length in a longitudinal direction to a first length in a transverse direction in each particle.

According to a prefered mode of the above first aspect, the powder has a BET specific surface area of 1 m²/g to 30 m²/g.

According to a prefered mode of the above first aspect, the powder is at least one of talc and/or mica.

According to a prefered mode of the above first aspect, a content of the powder relative to the mass of the composition is 0.2% by mass to 10% by mass.

According to a prefered mode of the above first aspect, a content of the cationic polymer relative to the mass of the composition is 0.00005% by mass to 0.5% by mass.

According to a prefered mode of the above first aspect, the cationic polymer further comprises 0.1% to 10% of a third component having a structure represented by undermentioned Chem. 4 relative to the molecular weight of the cationic polymer.

According to a prefered mode of the above first aspect, the cationic polymer further comprises 0.1% to 15% of a fourth component having a structure represented by undermentioned Chem. 6 relative to a molecular weight of the cationic polymer.

According to a prefered mode of the above first aspect, the cationic polymer is vinylpyrrolidone/N,N' -dimethylaminoethyl methacrylate/stearyl acrylate/tripropylene glycol diacrylate copolymer.

According to a prefered mode of the above first aspect, a content of a nonionic surfactant relative to the mass of the composition is 0.1% by mass or less.

According to a prefered mode of the above first aspect, a content of an oil component relative to the mass of the composition is 5% by mass or less.

According to a prefered mode of the above second aspect, the cationic polymer comprises, relative to a molecular weight of the cationic polymer, 15% to 85% of a first component having a structure represented by undermentioned Chem. 1, and 15% to 85% of a second component having a structure represented by Chem. 2 or Chem. 3.

According to a prefered mode of the above second aspect, the particle has an aspect ratio of two or greater. The aspect ratio is an average value of a second length in a longitudinal direction to a first length in a transverse direction.

According to a prefered mode of the above second aspect, the particle has a BET specific surface area of 1 m²/g to 30 m²/g.

According to a prefered mode of the above second aspect, the particle is at least one of talc and/or mica.

According to a prefered mode of the above second aspect, the cationic polymer further comprises, relative to a molecular weight of the cationic polymer, 0.1% to 10% of a third component having a structure represented by undermentioned Chem. 4.

According to a prefered mode of the above second aspect, the cationic polymer further comprises, relative to the molecular weight of the cationic polymer, 0.1% to 15% of a fourth component having a structure represented by Chem. 6.

According to a prefered mode of the above second aspect, the cationic polymer is vinylpyrrolidone/N,N'-dimethylaminoethyl methacrylate/stearyl acrylate/tripropylene glycol diacrylate copolymer.

In the following description, POE is an abbreviation of polyoxyethylene, and POP is an abbreviation of polyoxypropylene. The number in parentheses after POE or POP indicates the average number of moles of POE groups or POP groups added in the compound in question.

A powder-containing composition of the present disclosure according to a first embodiment and a method for manufacturing thereof are described.

The powder-containing composition of the present disclosure includes a powder, a cationic polymer, and an aqueous solvent.

### [Powder]

The powder bears a negative charge (minus charge) on its particle surface in the aqueous solvent to be described later. The negative charge may be ionic. The negative charge may be a charge charged by a charging agent or a coating agent. Analysis and evaluation for checking whether the powder bears the negative charge can be performed by measurement of an isoelectric point, measurement of pH and the like.

Examples of the powder bearing a negative charge on its surface in the aqueous solvent may include silica, talc, mica and the like.

The powder may be any powder as long as the cationic polymer can be adsorbed thereto in the aqueous solvent. Examples of other usable powders may include inorganic powder (such as kaolin, sericite, muscovite, phlogopite, synthetic mica, lepidolite, biotite, vermiculite, magnesium carbonate, calcium carbonate, aluminum silicate, barium silicate, calcium silicate, magnesium silicate, strontium silicate, tungstate, magnesium, silica, zeolite, glass, barium sulfate, calcined calcium sulfate (calcined gypsum), calcium phosphate, fluorine apatite, hydroxyapatite, ceramic powder, metallic soap (such as zinc myristate, calcium palimitate, and aluminum stearate), and boron nitride, etc); organic powder (such as polyamide resin powder (nylon powder), polyethylene powder, polymethylmethacrylate powder, polystyrene powder, styrene-acrylic acid copolymer powder, benzoguanamine resin powder, poly(tetrafluroethylene) powder, and cellulose powder, silicone resin powder, silk powder, wool powder, urethane powder, etc); inorganic white family pigment (such as titanium dioxide, zinc oxide, etc); inorganic red family pigment (such as iron oxide (colcothar), iron titanate, etc); inorganic brown family pigment (such as γ-iron oxide, etc); inorganic yellow family pigment (such as yellow iron oxide, loess, etc); inorganic black family pigment (such as black iron oxide, carbon black, lower titanium oxide, etc); inorganic purple family pigment (such as manganese violet, cobalt violet, etc); inorganic green family pigment (such as chrome oxide, chrome hydroxide, cobalt titanate, etc); inorganic blue family pigment (such as ultramarine, iron blue, etc); pearl pigment (such as titanium dioxide coated mica, titanium dioxide coated bismuth oxychloride, titanium dioxide coated talc, colored titanium dioxide coated mica, bismuth oxychloride, argentine, etc); metal powder pigment (such as aluminum powder, copper powder, etc); organic pigment such as zirconium, barium, or aluminum lake (such as organic pigment such as Red No.201, Red No.202, Red No.204, Red No.205, Red No.220, Red No.226, Red No.228, Red No.405, Red No.201, Orange No.203, Orange No.204, Yellow No.205, Yellow No.401, Blue No.401, Red No.3, Red No. 104, Red No. 106, Red No.227, Red No.230, Red No.401, Red No.505, Orange No.205, Yellow No.4, Yellow No.5, Yellow No.202, Yellow No.203, Green No.3, and Blue No.1, etc); natural pigment (such as chlorophyll, β-carotene, etc) and the like.

The powder may include a plurality of types of particles. The powder is preferably insoluble in the solvent.

The powder may be porous or non-porous. The particle in the powder may be in various forms. For example, the particle shape may be spherical, plate-shaped, needle-shaped, lump-shaped, indefinite-shaped, and the like. In the powder-containing composition of the present disclosure, a non-spherical (e.g. plate-shaped, lumps-shaped) and non-porous powder is preferred. Examples of the non-spherical non-porous powder may include talc and mica.

The powder may have a BET specific surface area of 0.5 m²/g or greater, 1 m²/g or greater, 1.5 m²/g or greater, or 2 m²/g or greater, for example. The powder may have a BET specific surface area of less than 50 m²/g, 30 m²/g or less, 20 m²/g or less, 10 m²/g or less, or 5 m²/g or less, for example. The BET specific surface area can be measured in accordance with JISZ8830, for example.

The powder may have an average particle size of 0.1 µm or greater, 0.5 µm or greater, 1 µm or greater, or 5 µm or greater, for example. The powder may have an average particle size of 100 µm or less, 70 µm or less, 50 µm or less, or 30 µm or less, for example. The average particle size of the powder can be measured in accordance with a dynamic light scattering method.

An average value of an aspect ratio of the powder may be an average value of a ratio of a second length in a longitudinal direction to a first length in a transverse direction (second length/first length) in each particle being 2 or greater, 2.5 or greater, 3 or greater, 5 or greater, 10 or greater, 20 or greater, or 50 or greater. The aspect ratio of the powder can be calculated by measuring the first lengths and second lengths of optional 100 particles in the powder by microscopic observation.

The content of the powder relative to the mass of the powder-containing composition may be 0.1% by mass or greater, 0.5% by mass or greater, 1% by mass or greater, or 5% by mass or greater. The content of the powder relative to the mass of the powder-containing composition may be 20% by mass or less, 15% by mass or less, 10% by mass or less, or 5% by mass or less.

### [Cationic polymer]

The cationic polymer is preferably soluble in the aqueous solvent such as water and alcohol. The cationic polymer may ionize under the presence of an acid. "Cationic" as used for the cationic polymer may mean that it becomes a cation by electrolytic dissociation when dissolved in the solvent, or becomes cationic under an acidic condition. Even in a latter case, the cationic polymer may not be cationized in the powder-containing composition. In the following description, each component is expressed with monomers for convenience.

The cationic polymer comprises a first component having a structure represented by the following Chem. 1. In Chem. 1, R¹ represents a hydrogen atom or a methyl group. R² and R³ each independently represent a hydrogen atom, a methyl group, an ethyl group, or a t-butyl group. A represents an oxygen atom or an NH group. B represents a C₁₋₄ alkylene group that is linear or has a side chain.

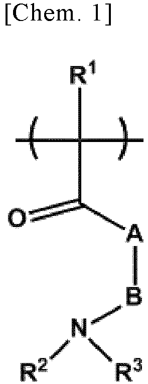

The first component is a component that acts to impart a property of a cationic ion to the cationic polymer when the polymer is processed with a suitable acid. The first component may be a (meth)acrylic acid derivative comprising an amine, for example. Examples of the first component may include 2-(dimethylamino)ethyl (meth)acrylate component, 3-(dimethylamino)propyl (meth)acrylate component, N-[2-(dimethylamino)ethyl](meth)acrylamide component, N-[3-(dimethylamino)propyl](meth)acrylamide component, and [N,N-dimethylaminoethyl (meth)acrylate component, N,N-diethylaminoethyl (meth)acrylate component, N,N-dimethylaminopropyl (meth)acrylamide component]. The first component may be a single type of component, or a combination of a plurality of types of components.

The mass percentage of the first component in the cationic polymer relative to the molecular weight of the cationic polymer (the total amount of the first to fourth components) is preferably 15% or greater, more preferably 25% or greater, and further more preferably 30% or greater. If the first component is less than 15%, it may become difficult for the cationic polymer to adhere to the powder surface. The mass percentage of the first component in the cationic polymer relative to the molecular weight of the cationic polymer (the total amount of the first to fourth components) is preferably 85% or less, more preferably 75% or less, more preferably 65% or less, more preferably 60% or less, and further more preferably 55% or less. If the first component exceeds 85%, a complex formed from the cationic polymer and the powder may easily aggregate with each other.

The cationic polymer has a second component having a structure represented by the following Chem. 2 or Chem. 3. In Chem. 2 and Chem. 3, R⁴ represents a hydrogen atom or a methyl group. In Chem. 2, p represents 3 or 4.

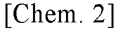

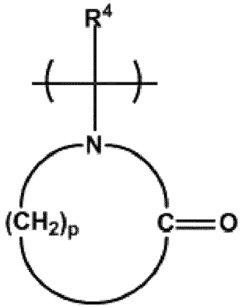

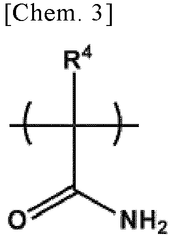

Examples of the heterocycle in the second component may include lactams (cyclic amides). Examples of lactams may include three-membered rings, four-membered rings, five-membered rings (pyrrolidones), and six-membered rings (piperidones). The heterocycle is preferably provided at a terminal of a side chain. Examples of the second component may include N-vinylpyrrolidone component, N-vinylpiperidone component, acrylamide component, and methacrylamide component. The second component may be a single type of component, or a combination of a plurality of types of components.

The mass percentage of the second component in the cationic polymer relative to the molecular weight of the cationic polymer (the total amount of the first to fourth components) is preferably 5% or greater, more preferably 10% or greater, more preferably 15% or greater, more preferably 25% or greater, and further more preferably 30% or greater. If the second component is less than 5%, a complex formed from the cationic polymer and the powder may aggregate easily. The mass percentage of the second component in the cationic polymer relative to the molecular weight of the cationic polymer (the total amount of the first to fourth components) is preferably 85% or less, more preferably 75% or less, more preferably 65% or less, more preferably 60% or less, and further more preferably 55% or less. If the second component exceeds 85%, it may become difficult for the cationic polymer to adhere to the powder surface.

Examples of the cationic polymer including the first and second components may include N-vinylpyrrolidone/N,N-dimethylaminoethyl methacrylate copolymer.

The cationic polymer is preferably a copolymer that further has a third component having a structure represented by the following Chem. 4. In Chem. 4, R⁵ represents a hydrogen atom or a methyl group. D represents an oxygen atom or an NH group. R⁶ represents a C₁₋₁₇ alkylene group that is linear or has a side chain, or a group represented by Chem. 5. R⁷ represents a hydrogen atom or a methyl group. In Chem. 5, n represents an integer of 1 to 4, and q represents an integer of 1 to 25.

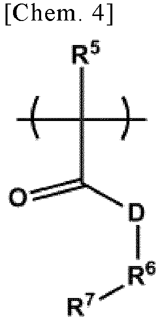

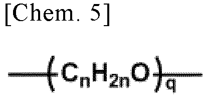

The third component may include a (meth)acryloyl group. Examples of the third component may include alkyl (meth)acrylates and alkyl (meth)acrylamides. The number of carbon atoms in the alkyl group may be one or greater, five or greater, eight or greater, or ten or greater, for example. The number of carbon atoms in the alkyl group may be 50 or less, 40 or less, 30 or less, or 20 or less, for example. Examples of the third component may include methyl (meth)acrylate component, ethyl (meth)acrylate component, n-propyl (meth)acrylate component, isopropyl (meth)acrylate component, n-butyl (meth)acrylate component, isobutyl (meth)acrylate component, t-butyl (meth)acrylate component, n-hexyl (meth)acrylate component, 2-ethylhexyl (meth)acrylate component, octyl (meth)acrylate component, lauryl (meth)acrylate component, tridecyl (meth)acrylate component, stearyl (meth)acrylate component, cyclohexyl (meth)acrylate component, N-t-butyl (meth)acrylamide component, N-t-octyl (meth)acrylamide component, hydroxy ethyl (meth)acrylate component, hydroxypropyl (meth)acrylate component, and hydroxybutyl (meth)acrylate component. The third component may be a single type of component, or a combination of a plurality of types of components.

The mass percentage of the third component in the cationic polymer relative to the molecular weight of the cationic polymer (the total amount of the first to fourth components) may be 0%, preferably 0.5% or greater, more preferably 1% or greater, and further more preferably 1.2% or greater. The mass percentage of the third component in the cationic polymer relative to the molecular weight of the cationic polymer (the total amount of the first to fourth components) is preferably 60% or less, more preferably 30% or less, more preferably 10% or less, and further more preferably 5% or less. If the third component exceeds 60%, it may become difficult for the cationic polymer to adhere to the powder surface.

The cationic polymer is preferably a copolymer that further has a fourth component having a structure represented by the following Chem. 6. In Chem. 6, R⁸ represents a hydrogen atom or a methyl group. E represents an oxygen atom or an NH group. R⁹ represents a C₁₋₁₇ alkylene group that is linear or has a side chain, or a group represented by Chem. 7. R¹⁰ represents a hydrogen atom or a methyl group. In Chem. 7, n represents an integer of 1 to 4, and r represents an integer of 1 to 25.

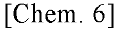

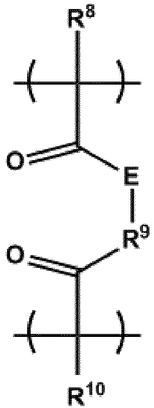

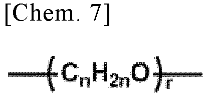

The fourth component may be a crosslinking component. For example, a compound having two or more carbon-carbon unsaturated double bonds in one molecule may be used as the fourth component. For example, the fourth component may be a monomer having vinyl groups at both terminals.

The fourth component may have a hydrophilic moiety. The hydrophilic moiety is preferably located between the two double bonds that contribute to polymerization. The hydrophilic moiety may be a polyoxyalkylene chain such as a polyoxyethylene chain or a polyoxypropylene chain, for example. The average number of moles of polyoxyethylene groups and/or polyoxypropylene groups added may be one or greater, and preferably two or greater, for example. The average number of moles of polyoxyethylene groups and/or polyoxypropylene groups added may be ten or less, and preferably five or less, for example.

Examples of the fourth component may include: (poly)ethylene glycol di(meth)acrylate component; (poly)propylene glycol di(meth)acrylate component; trimethylolpropane tri(meth)acrylate component; pentaerythritol tri(meth)acrylate component; methylenebis(meth)acrylamide; 1,2-bis(meth)acrylamide ethane component; 1,5-bis(meth)acrylamide pentane component; and divinylbenzene component. The fourth component may be a single type of component, or a combination of a plurality of types of components.

The mass percentage of the fourth component in the cationic polymer relative to the molecular weight of the cationic polymer (the total amount of the first to fourth components) may be 0%, preferably 0.1% or greater, more preferably 1% or greater, more preferably 3% or greater, and further more preferably 4% or greater. The mass percentage of the fourth component in the cationic polymer relative to the molecular weight of the cationic polymer (the total amount of the first to fourth components) is preferably 20% or less, more preferably 15% or less, more preferably 10% or less, and further more preferably 8% or less. If the fourth component exceeds 20%, it may become difficult for the cationic polymer to adhere to the powder surface.

The cationic polymer may comprise components other than the first to fourth components.

Examples of the cationic polymer including the first to fourth components may include Polyacrylate-1. As Polyacrylate-1, for example, vinylpyrrolidone/N,N'-dimethylaminoethyl methacrylate/stearyl acrylate/tripropylene glycol diacrylate copolymer (Cosquat GA468; manufactured by OSAKA ORGANIC CHEMICAL INDUSTRY LTD.) may be used.

The weight-average molecular weight of the cationic polymer is preferably 100,000 or greater, more preferably 500,000 or greater, and further more preferably 1,000,000 or greater. If the weight-average molecular weight is less than 100,000, it may become difficult to suppress aggregation of the powder. The weight-average molecular weight of the cationic polymer is preferably 5,000,000 or less, more preferably 3,000,000 or less, and further more preferably 2,000,000 or less. If the weight-average molecular weight exceeds 5,000,000, clogging and stickiness may occur. The weight-average molecular weight of the cationic polymer can be measured with a gel permeation chromatography (GPC-MALS) by a light scattering method.

The addition amount of the cationic polymer relative to the total surface area 1 m² of the powder is preferably 1 µg or greater, more preferably 3 µg or greater, more preferably 5 µg or greater, more preferably 10 µg or greater, and further more preferably 15 µg or greater. If the addition amount of the cationic polymer is less than 1 µg, dispersibility of the powder cannot be enhanced. The addition amount of the cationic polymer relative to the total surface area 1 m² of the powder is preferably 10,000 µg or less, more preferably 7,000 µg or less, and further more preferably 6,000 µg or less. If the addition amount of the cationic polymer exceeds 10,000 µg, aggregates of the powder may be formed easily, and dispersibility may deteriorate. The addition amount of the cationic polymer to the total surface area 1 m² of the powder may be 5,000 µg or less, 3,000 µg or less, 1,000 µg or less, 700 µg or less, or 500 µg or less.

With respect to the cationic polymer, matters described in Japanese Unexamined Patent Publication No. H6-219921 A may be incorporated herein by reference.

The cationic polymer is considered to be adhered to the particle surface in the aqueous solvent. In a case where a secondary particle is formed, the cationic polymer is considered to be adhered to the surface of the secondary particle. In the present disclosure, the term "particle" denotes a minimum unit that can be dispersed, and may include primary and secondary particles. It is considered that a direct contact between the particles is prevented by the cationic polymer adsorbed to the particle surface, so that aggregation and solidification of the powder are suppressed. Moreover, it is considered that the particles are adjacent to each other via the cationic polymer, so that redispersibility of the powder is enhanced by repulsion between the cationic polymers.

### [Aqueous solvent]

The aqueous solvent is preferably water and/or a solvent that has a high affinity with water. Examples of the aqueous solvent may include water, water-soluble alcohol, or a mixture thereof.

With respect to water, water used for such as cosmetics and quasi-pharmaceutical products can be used, including e.g., purified water, ion-exchanged water, and tap water.

Examples of the lower alcohol may include ethanol, propanol, isopropanol, isobutyl alcohol, t-butyl alcohol, and the like.

Examples of the polyhydric alcohol may include dihydric alcohol (such as ethylene glycol, propylen glycol, trimethylene glycol, 1,2-butylene glycol, 1,3-butylene glycol, tetramethylene glycol, 2,3-butylene glycol, pentamethylene glycol, 2-butene-1,4-diol, hexylene glycol, octylene glycol, etc); trihydric alcohol (such as glycerin, trimethylolpropane, etc); tetrahydric alcohol (such as such as pentaerythritol such as 1,2,6-hexanetriol, etc); pentahydric alcohol (such as xylitol, etc); hexahydric alcohol (such as sorbitol, mannitol, etc); polyhydric alcohol polymer (such as diethylene glycol, dipropylene glycol, triethylene glycol, polypropylene glycol, tetraethylene glycol, diglycerin, polyethylene glycol, triglycerin, tetraglycerin, polyglycerin, etc); dihydric alcohol alkyl ethers (such as ethylene glycol monomethyl ether, ethylene glycol monoethyl ether, ethylene glycol monobutyl ether, ethylene glycol monomphenyl ether, ethylene glycol monohexyl ether, ethylene glycol mono2-methylhexyl ether, ethylene glycol isoamyl ether, ethylene glycol benzil ether, ethylene glycol isopropyl ether, ethylene glycol dimethyl ether, ethylene glycol diethyl ether, ethylene glycol dibutyl ether, etc); dihydric alcohol alkyl ethers (such as diethylene glycol monomethyl ether, diethylene glycol monoethyl ether, diethylene glycol monombutyl ether, diethylene glycol dimethyl ether, diethylene glycol diethyl ether, diethylene glycol butyl ether, diethylene glycol methylethyl ether, triethylene glycol monomethyl ether, triethylene glycol monoethyl ether, propylene glycol monomethyl ether, propylene glycol monoethyl ether, propylene glycol monobutyl ether, propylene glycol isopropyl ether, dipropylene glycol methyl ether, dipropylene glycol ethyl ether, dipropylene glycol butyl ether, etc); dihydric alcohol ether ethers (such as ethylene glycol monomethyl ether acetate, ethylene glycol monoethyl ether acetate, ethylene glycol monobutyl ether acetate, ethylene glycol monophenyl ether acetate, ethylene glycol diadipate, ethylene glycol disaccinate, diethylene glycol monoethyl ether acetate, diethylene glycol monobutyl ether acetate, propylene glycol monomethyl ether acetate, propylene glycol monoethyl ether acetate, propylene glycol monopropyl ether acetate, propylene glycol monophenyl ether acetate, etc); glycerin monoalkyl ether (such as chimyl alcohol, selachyl alcohol, batyl alcohol, etc); sugar alcohol (such as sorbitol, maltitol, maltotriose, mannitol, sucrose, erythritol, glucose, fructose, starch sugar, maltose, xylitol, starch sugar hydrogenated alcohol, etc); glycolide, tetrahydrofurfuryl alcohol; POE-tetrahydrofurfuryl alcohol; POP/POE-butyl ether; tripolyoxypropylene glycerin ether; POP-glycerin ether; POP-glycerin ether phosphoric acid; POP/POE-pentaerythritol ether; polyglycerin, and the like.

The content of the aqueous solvent relative to the mass of the powder-containing composition may be 30% by mass or greater, 40% by mass or greater, 50% by mass or greater, 60% by mass or greater, 70% by mass or greater, 80% by mass or greater, or 90% by mass or greater, for example. The content of water in the aqueous solvent relative to the mass of the aqueous solvent may be 40% by mass or greater, 50% by mass or greater, 60% by mass or greater, 70% by mass or greater, 80% by mass or greater, or 90% by mass or greater.

### [Nonionic surfactant]

The powder-containing composition of the present disclosure may include a nonionic surfactant. However, the powder-containing composition of the present disclosure does not need to include the nonionic surfactant for achieving the effect of the present disclosure.

The content of the nonionic surfactant relative to the mass of the powder-containing composition may be less than 0.1% by mass, less than 0.05% by mass, or substantially 0% by mass.

The ratio of the mass of the nonionic surfactant to the mass of the cationic polymer in the powder-containing composition ([mass of nonionic surfactant]/[mass of cationic polymer]) may be less than one, less than 0.8, less than 0.5 or substantially zero.

Examples of the lipophilic nonionic surfactants may include sorbitan fatty acid ester (such as sorbitan monooleate, sorbitan monoisostearate, sorbitan monolaurate, sorbitan monopalmitate, sorbitan monostearate, sorbitan sesquioleate, sorbitan trioleate, diglycerol sorbitan penta-2 ethylhexylate, diglycerol sorbitan tetra-2 ethylhexylate, etc); glyceryl polyglyceryl fatty acid (such as glyceryl monocotton oil fatty acid, glyceryl monoerucate, glyceryl sesquioleate, glyceryl monostearate, glyceryl α, α'-oleate pyroglutamate, glyceryl monostearate malate, etc); propylene glycol fatty acid ester (such as propylene glycol monostearate, etc); hydrogenated caster oil derivative; glyceryl alkyl ether, and the like.

Examples of the hydrophilic nonionic surfactants that may be used may include POE sorbitan fatty acid ester (such as POE sorbitan monooleate, POE sorbitan monostearate, POE sorbitan monooleate, POE sorbitan tetraoleate); POE sorbit fatty acid ester (such as POE sorbit monolaurate, POE sorbit monooleate, POE sorbit pentaoleate, POE sorbit monostearate), POE glyceryl fatty acid ester (such as POE monooleate such as POE glyceryl monostearate, POE glyceryl monoisostearate, POE glyceryl triisostearate); POE fatty acid ester (such as POE distearate, POE monodioleate, ethyleneglycol distearate); POE alkyl ether (such as POE lauryl ether, POE oleyl ether, POE stearyl ether, POE behenyl ether, POE-2-octyldodecyl ether, POE cholestanol ether); puluronic type (such as Puluronic), POE/POP alkyl ethers (such as POE/POP cetyl ether, POE/POP 2-decyltetradecyl ether, POE/POP monobutyl ether, POE/POP hydrogenated lanoline, POE/POP glycerin ether); tetra POE/tetra POP ethylenediamine condensation products (such as Tetronic); POE castor oil hydrogenated castor oil derivative (such as POE caster oil, POE hydrogenated caster oil, POE hydrogenated caster oil monoisostearate, POE hydrogenated castor oil triisostearate, POE hydrogenated caster oil monopyroglutamate monoisostearate diester, POE hydrogenated oil maleate); POE beeswax/lanoline derivative (such as POE sorbitol beeswax); alkanolamide (such as coconut oil fatty acid diethanolamide, lauric acid monoethanolamide, fatty acid isopropanolamide); POE propyleneglycol fatty acid ester; POE alkyl amines; POE fatty acid amide; sucrose fatty acid ester; alkylethoxydimethylamine oxide; trioleyl phosphoric acid and the like.

### [Oily component]

In the powder-containing composition of the present disclosure, the oily component relative to the mass of the powder-containing composition may be 5% by mass or less, 3% by mass or less, 1% by mass or less, or substantially 0% by mass. By lowering the content of the oily component, stickiness that occurs when the composition is applied to skin can be suppressed.

Examples of the oily component that may be used include liquid oils, solid fats, waxes, hydrocarbons, higher fatty acids, higher alcohols, synthetic ester oils, and silicone oils.

Examples of the liquid oil that may be used may include avocado oil, camellia oil, turtle oil, macadamia nut oil, corn oil, mink oil, olive oil, rapeseed oil, egg yolk oil, sesame oil, par chic oil, wheat germ oil, southern piece oil, castor oil, linseed oil, safflower oil, cotton seed oil, perilla oil, soybean oil, groundnut oil, brown real oil, torreya oil, rice bran oil, Chinese tung oil, Japanese tung oil, jojoba oil, germ oil, triglycerol, and the like.

Examples of the solid fat that may be used may include cacao butter, coconut oil, horse fat, hydrogenated coconut oil, palm oil, beef tallow, sheep tallow, hydrogenated beef tallow, palm kernel oil, lard, beef bones fat, Japan wax kernel oil, hardened oil, hoof oil, Japan wax, hydrogenated caster oil, and the like.

Examples of the waxes that may be used may include beeswax, candelilla wax, cotton wax, carnauba wax, bayberry wax, insect wax, spermaceti, montan wax, bran wax, lanolin, kapok wax, lanolin acetate, liquid lanolin, sugarcane wax, lanolin fatty acid isopropyl ester, hexyl laurate, reduced lanolin, jojoba wax, hardened lanolin, shellac wax, POE lanolin alcohol ether, POE lanolin alcohol acetate, POE cholesterol ether, lanolin fatty acid polyethylene glycol, POE hydrogenated lanolin alcohol ether, and the like.

Examples of the hydrocarbon oils that may be used may include liquid paraffin, ozocerite, squalane, pristane, paraffin, ceresin. squalene, vaseline, microcrystalline wax, isododecane, isohexadecane, and the like.

Examples of the higher fatty acid that may be used may include lauric acid, myristic acid, palmitic acid, stearic acid, behenic acid, oleic acid, undecylenic acid, tallic acid, isostearic acid, linoleic acid, linolenic acid, eicosapentaenoic acid(EPA), docosahexaenoic acid(DHA) and the like.

Examples of the higher alcohol that may be used may include linear alcohol (such as lauryl alcohol, cetyl alcohol, stearyl alcohol, behenyl alcohol, myristyl alcohol, oleyl alcohol, and cetostearyl alcohol); branched-chain alcohol (such as monostearylglycerin ether (batyl alcohol), 2-decyltetradecinol, lanolin alcohol, cholesterol, phytosterol, hexyldodecanol, isostearyl alcohol, and octyldodecanol) and the like.

Examples of the synthesis ester oils that may be used may include isopropyl myristate, cetyl octanoate, octyldodecyl myristate, isopropyl palmitate, butyl stearate, hexyl laurate, myristyl myristate, decyl oleate, hexyldecyl dimethyl octanoate, cetyl lactate, myristyl lactate, lanolin acetate, isocetyl stearate, isocetyl isostearate, cholesteryl 12-hydroxy stearate, ethylene glycol di-2-ethyl hexanoate, di-penta erythritol fatty acid ester, N-alkyl glycol monoisostearate, neopentyl glycol dicaprate, diisostearyl malate, glyceryl di-2-heptyl undecanoate, trimethyrol propane tri-2-ethyl hexanoate, trimethyrol propane triisostearate, pentaerythritol tetra-2-ethyl hexanoate, glyceryl tri-2-ethyl hexanoate, glyceryl trioctanoate, glyceryl triisopalmitate, trimethyrol propane triisostearate, cetyl 2-ethylhexanoate, 2-ethylhexyl palmitate, glyceryl trimyristate, glyceride tri-2-heptyl undecanoate, castor oil fatty acid methyl ester, oleyl oleate, acetoglyceride, 2-heptylundecyl palmitate, diisobutyl adipate, N-lauroyl-L-glutamic acid-2-octyldodecyl ester, di-2-heptylundecyl adipate, ethyl laurate, di-2-ethylhexyl sebacate, 2-hexyldecyl myristate, 2-hexyldecyl palmitate, 2-hexyldecyl adipate, diisopropyl sebacate, 2-ethylhexyl succinate, triethyl citrate, and the like.

Examples of the silicone oil may include silicone compounds such as dimethylpolysiloxane, methylhydrogenpolysiloxane, methylphenylpolysiloxane, stearoxymethylpolysiloxane, polyether-modified organopolysiloxane, fluoroalkyl/polyoxyalkylene co-modified organopolysiloxane, alkyl-modified organopolysiloxane, terminal-modified organopolysiloxane, fluorine-modified organopolysiloxane, amino-modified organopolysiloxane, silicone gel, acrylic silicone, trimethylsiloxysilicic acid, silicone RTV rubber and the like.

### [Other components]

The composition of the present disclosure may include, as appropriate and as necessary, other components such as monosaccharides, oligosaccharides, polysaccharides and derivatives thereof; esters, anionic surfactants, cationic surfactants, amphoteric surfactants, moisturizers, water-soluble polymers, thickeners, film-forming agents, ultraviolet light absorbers, sequestrants, amino acids, organic amines, polymeric emulsions, pH adjusters, skin nutrients, vitamins, antioxidants, antioxidant aids, perfumes, and water in amounts that do not inhibit the effects of the present disclosure.

Examples of other components that may be blended are listed below. At least one of the following components may be added to the composition of the present disclosure.

Examples of the monosaccharides may include at least one selected from triose (such as D-glyceryl aldehyde, dihydroxyacetone, etc); tetrose (such as D-erythrose, D-erythrulose, D-threose, erythritol, etc); pentaose (such as L-arabinose, D-xylose, L-lyxose, D-arabinose, D-ribose, D-ribulose, D-xylulose, L-xylulose, etc); hexalose (such as D-glucose, D-talose, D-psicose, D-galactose, D-fructose, L-galactose, L-mannose, D-tagatose, etc); heptose (such as aldoheptose, heptulose, etc); octose (such as octulose, etc); deoxy sugar (such as 2-deoxy-D-ribose, 6-deoxy-L-galactose, 6-deoxy-L-mannose, etc); amino sugar (such as D-glucosamine, D-galactosamine, sialic acid, amino uronic acid, muramic acid, etc); uronic acid (such as D-grucuronic acid, D-mannuronic acid, L-guluronic acid, D-garacturonic acid, L-iduronic acid, etc) and the like.

Examples of the oligosaccharide may include at least one selected from sucrose, guntianose, umbelliferose, lactose, planteose, isolignoses, α,α-trehalose, raffinose, lignoses, umbilicin, stachyose, verbascoses, and the like.

Examples of the polysaccharide may include at least one selected from cellulose, quince seed, chondroitinsulfate, starch, galactan, dermatan sulfate, glycogen, acasia gum, heparansulfate, hyaluronan, gum tragacanth, keratan sulfate, chondoroitin, xanthan gum, mucoitin sulfate, guar gum, dextran, keratosulfate, locust bean gum, succinoglycan, caronic acid, and the like.

Examples of other polyols may include at least one polyol selected from polyoxyethylene methyl glucoside (Glucam E-10), polyoxypropylene methyl glucoside (Glucam P-10), and the like.

Examples of the anionic surfactants that may be used may include fatty acid soap (such as sodium laurate, and sodium palmitate); higher alkyl sulfate ester salt (such as sodium lauryl sulfate, and potassium lauryl sulfate); alkyl ether sulfate ester salt (such as POE-lauryl sulfate triethanolamine, and sodium POE-lauryl sulfate); N-acyl sarcosinic acid (such as sodium lauroyl sarcocinate); higher fatty acid amide sulfonate (such as sodium N-stearoyl-N-methyltaurate, sodium N-myristoyl-N-methyltaurate, sodium methyl cocoyl taurate, and sodium laurylmethyl taurate); phosphate ester salt (sodium POE-oleylether phosphate, POE-stearylether phosphate, potassium cetyl phosphate); sulfosuccinate (such as sodium di-2-ethylhexyl sulfosuccinate, sodium monolauroyl monoethanolamide polyethylene sulfosuccinate, and sodium lauryl polypropylene glycol sulfosuccinate); alkylbenzene sulfonate (such as sodium linear dodecylbenzene sulfonate, triethanolamine linear dodeylbenzene sulfonate, and linear dodecylbenzene sulfonate); higher fatty acid ester sulfate ester salt (such as sodium hydrogenated gryceryl cocoate sulfate); N-acyl glutamate (such as monosodium N-lauroyl glutamate, disodium N-stearoyl glutamate, and monosodium N-myristoyl-L-glutamate); sulfonated oil (such as Turkey red oil); POE-alkyl ether carboxylic acid; POE-alkyl aryl ether carboxylate; α-olefine sulfonate; higher fatty acid ester sulfonate; secondary alcohol sulfate ester salt; higher fatty acid alkylolamide sulfate ester salt; sodium lauroyl monoethanolamide succinate; N-palmitoyl asparaginate ditriethanolamine; sodium casein; and the like.

Examples of the cationic surfactants may include alkyltrimethyl ammonium salt (such as stearyltrimethyl ammonium chloride, lauryltrimethyl ammonium chloride); alkylpyridinium salt (such as cetylpyridinium chloride); dialkyldimethyl ammonium salt (such as distearyldimethyl ammonium chloride); poly (N,N'-dimethyl-3,5-methylenepiperidinium) chloride; alkyl quaternary ammonium salt; alkyldimethylbenzyl ammonium salt; alkylisoquinolinium salt; dialkylmorphonium salt; POE alkylamine; alkylamine salt; polyamine fatty acid derivative; amyl alcohol fatty acid derivative; benzalkonium chloride; benzethonium chloride, and the like.

Examples of the amphoteric surfactant that may be used may include: imidazoline-based amphoteric surfactant (such as sodium 2-undecyl-N,N,N-(hydroxyethylcarboxymethyl)-2-imidazoline and 2-cocoyl-2-imidazolinium hydroxide-1-carboxyethyloxy disodium salt); and betaine-based surfactant (such as 2-heptadecyl-N-carboxymethyl-N-hydroxyethyl imidazolinium betaine, lauryl dimethylaminoacetic acid betaine, alkyl betaine, amidobetaine, and sulfobetaine).

Examples of the moisturizers may include polyethylene glycol, propylene glycol, glycerin, 1,3-butylene glycol, xylitol, sorbitol, maltitol, chondroitin sulfate, hyaluronic acid, mucoitin sulfate, charonic acid, atelocollagen, cholesteryl 12-hydroxystearate, sodium lactate, bile salt, dl-pyrrolidone carboxylate, alkyleneoxide derivative, short-chain soluble collagen, diglycerin (EO)PO adduct, chestnut rose extract, yarrow extract, melilot extract, and the like.

Examples of the natural water-soluble polymer may include plant-based polymer (such as gum Arabic, gum tragacanth, galactan, guar gum, locust bean gum, gum karaya, carrageenan, pectine, agar, quince seed (cydonia oblonga), algae colloid (brown algae extract), starch (rice, corn, potato, wheat), glicyrrhizic acid); microorganism based polymer (such as xanthan gum, dextran, succinoglycan, pullulan, etc), animal-based polymer (such as collagen, casein, albumin, gelatine, etc) and the like.

Examples of the semisynthetic water-soluble polymer may include starch-based polymer (such as carboxymethyl starch, methylhydroxypropyl starch, etc); cellulose-based polymer (such as methylcellulose, ethylcellulose, methylhydroxypropylcellulose, hydroxyethylcellulose, cellulose sodium sulfate, hydroxypropylcellulose, carboxymethylcellulose, sodium calboxymethyl cellulose, crystalline cellulose, cellulose powder, etc); algin acid-based polymer (such as sodium alginate, propylene glycol alginate ester, etc), and the like.

Examples of the synthetic water-soluble polymer may include vinyl based polymer (such as polyvinyl alcohol, polyvinyl methyl ether, polyvinylpyrrolidone, carboxyvinylpolymer, etc); polyoxyethylene based polymer (such as polyoxyethylenepolyoxypropylene copolymer such as polyethylene glycol 20,000, 40,000 and 60,000, etc); acrylic polymer (such as sodium polyacrylate, polyethylacrylate, polyacrylamide, etc); polyethyleneimine; cationic polymer; and the like.

Examples of thickeners may include gum arabic, carrageenan, karaya gum, tragacanth gum, carob gum, quince seed (marmelo), casein, dextrin, gelatin, sodium pectate, sodium alginate, methyl cellulose, ethyl cellulose, carboxymethyl cellulose (CMC), hydroxyethyl cellulose, hydroxypropyl cellulose, polyvinyl alcohol (PVA), polyvinylmethyl ether (PVM), PVP (polyvinyl pyrrolidone), polysodium acrylate, carboxyvinyl polymer, locust bean gum, guar gum, tamarind gum, dialkyldimethylammonium sulfate cellulose, xanthan gum, aluminum magnesium silicate, bentonite, hectorite, aluminum magnesium silicate (Veegum), sodium magnesium silicate (Laponite), silicic acid anhydride, and the like.

Examples of the film-forming agent may include an anionic film-forming agent (such as (meta)acrylic acid/(meta)acrylic acid ester copolymer, methyl vinyl ether/maleic anhydride coplymer, etc), a cationic film-forming agent (such as cationic cellulose, diallyldimethylammonium chloride polymer, diallyldimethylammonium chloride/acrylic amide copolymer, etc), a nonionc film-forming agent (such as polyvinyl alcohol, polyvinylpyrrolidone, polyvinyl acetate, polyacrylic ester copolymer, (meta)acrylamide, polymeric silicone, silicone resin, trimethylsiloxysilicate, etc), and the like.

Examples of the ultraviolet light absorbers may include benzoic acid family ultraviolet light absorber (such as p-aminobenzoic acid (hereinafter abbreviated as PABA), PABA monoglycerine ester, N,N-dipropoxy PABA ethyl ester, N,N-diethoxy PABA ethyl ester, N,N-dimethyl PABA ethyl ester, N,N-dimethyl PABA butyl ester, N,N-dimethyl PABA ethyl ester, etc); anthranilic acid family ultraviolet light absorber (such as homomenthyl N-acetylanthranilate etc); salicylic acid family ultraviolet light absorber (such as amyl salicylate, menthyl salicylate, homomenthyl salicylate, octyl salicylate, phenyl salicylate, benzyl salicylate, p-isopropanolphenyl salicylate, etc); cinnamic acid family ultraviolet light absorber (such as octyl methoxycinnamate, ethyl 4-isopropylcinnamate, methyl 2,5-diisopropylcinnamate, ethyl 2,4-diisopropylcinnamate, methyl 2,4-diisopropylcinnamate, propyl p-methoxycinnamate, isopropyl p-methoxycinnamate, isoamyl p-methoxycinnamate, octyl p-methoxycinnamate (2-ethylhexyl p-methoxycinnamate), 2-ethoxyethyl p-methoxycinnamate, cyclohexyl p-methoxycinnamate, ethyl α-cyano-β-phenylcinnamate, 2-ethylhexyl α-cyano-β-phenylcinnamate, glyceryl mono-2-ethylhexanoyl-diparamethoxy cinnamate, etc); benzophenone family ultraviolet light absorber (such as 2,4-dihydroxybenzophenone, 2,2'-dihydroxy-4-methoxybenzophenone, 2,2'-dihydroxy-4,4'-dimethoxybenzophenone, 2,2',4,4'-tetrahydroxybenzophenone, 2-hydroxy-4-methoxybenzophenone, 2-hydroxy-4-methoxy-4'-methylbenzophenone, 2-hydroxy-4-methoxybenzophenone-5-sulfonate, 4-phenylbenzophenone, 2-ethylhexyl-4'-phenyl-benzophenone-2-carboxylate, 2-hydroxy-4-n-octoxybenzophenone, 4-hydroxy-3-carboxybenzophenone, etc); 3-(4'-methylbenzylidene)-d,1-camphor and 3-benzylidene-d,1-camphor; 2-phenyl-5-methylbenzoxazol; 2,2'-hydroxy-5-methylphenylbenzotriazol, 2-(2'-hydroxy-5'-t-octylphenyl) benzotriazol, 2-(2'-hydroxy-5'-methylphenylbenzotriazol; dibenzalazine; dianisoylmethane; 4-methoxy-4'-t-butyldibenzoylmethane; 5-(3,3-dimethyl-2-norbornylidene)-3-pentane-2-one; dimorpholinopyridazinone; 2-ethylhexyl 2-cyano-3,3-diphenylacrylate; 2,4-bis-{[4-(2-ethylhexyloxy)-2-hydroxy]-phenyl}-6-(4-methoxyphenyl)-(1,3,5)-triazine, and the like.

Examples of the metal ion sequestrant may include 1-hydroxyethane-1, 1-diphosphonic acid, 1-hydroxyethane, 1-diphosphonic acid 4Na salt, disodium edetate, trisodium edetate, tetrasodium edetate, sodium citrate, sodium polyphosphate, sodium metaphosphate, gluconic acid, phosphoric acid, citric acid, ascorbic acid, succinic acid, edetic acid, trisodium hydroxyethyl ethylenediamine triacetate, and the like.

Examples of the amino acid may include neutral amino acid (such as threonine, cysteine, etc); basic amino acid (such as hydroxylysine, etc) and the like. Examples of the amino acid derivative may include sodium acyl sarcosinate (sodium lauroyl sarcosinate), acyl glutamate, sodium acyl β-alanine, glutathione, pyrrolidone carboxylate, and the like.

Examples of the organic amine may include monoethanolamine, diethanolamine, triethanolamine, morpholine, triisopropanolamine, 2-amino-2-methyl-1,3-propanediol, 2-amino-2-methyl-1-propanol, and the like.

Examples of the polymer emulsion may include acrylic resin emulsion, ethyl polyacrylate emulsion, solution of acrylic resin, polyacrylalkylester emulsion, polyvinyl acetate resin emulsion, natural rubber latex, and the like.

Examples of the pH modifier may include buffer such as lactic acid-sodium lactate, citric acid-sodium citrate, succinic acid-sodium succinate, and the like.

Examples of the vitamins may include vitamine A, B1, B2, B6, C, E and derivatives thereof, pantothenic acid and derivatives thereof, biotin, and the like.

Examples of the anti-oxidant may include tocopherols, dibutyl hydroxy toluene, butyl hydroxy anisole, and gallic acid esters, and the like.

Examples of the anti-oxidant aid may include phosphoric acid, citric acid, ascorbic acid, maleic acid, malonic acid, succinic acid, fumaric acid, cephalin, hexamethaphosphate, phytic acid, ethylenediaminetetraacetic acid, and the like.

Examples of other containable compositions may include an antiseptic agent (such as ethylparaben, butylparaben, chlorphenesin, 2-phenoxyethanol, etc); antiphlogistic (such as glycyrrhizinic acid derivatives, glycyrrhetic acid derivatives, salicylic acid derivatives, hinokitiol, zinc oxide, allantoin, etc); a skin-whitening agent (such as placental extract, saxifrage extract, arbutin, etc); various extracts (such as phellodendron bark (cork tree bark), coptis rhizome, lithospermum, peony, swertia herb, birch, sage, loquat, carrot, aloe, mallow, iris, grape, coix seed, sponge gourd, lily, saffron, cnidium rhizome, ginger, hypericum, restharrow, garlic, red pepper, citrus unshiu, Japanese angelica, seaweed, etc); an activator (such as royal jelly, photosenstizer, cholesterol derivatives, etc); a blood circulation promotion agent (such as nonylic acid vanillylamide, nicotine acid benzyl ester, nicotine acid β-butoxyethyl ester, capsaicin, zingerone, cantharides tincture, ichthammol, tannic acid, α-borneol, tocopheryl nicotinate, meso-inositol hexanicotinate, cyclandelate, cinnarizine, tolazoline, acetylcholine, verapamil, cepharanthine, γ-oryzanol, etc); an antiseborrheric agent, (such as sulfur, thianthl, etc); an anti-inflammatory agent (such as tranexamic acid, thiotaurine, hypotaurine, etc), and the like.

The composition of the present disclosure further may inculde, as necessary, caffeine, tannin, verapamil, tranexamic acid and derivatives thereof; various crude drug extracts such as licorice, Chinese quince, Pyrola japonica and the like; drugs such as tocopherol acetate, glycyrrhetinic acid, glycyrrhizic acid and derivatives thereof, or salts thereof; skin-whitening agents such as vitamin C, magnesium ascorbyl phosphate, ascorbic acid glucoside, arbutin, kojic acid and the like; amino acids such as arginine and lysine and the like and derivatives thereof.

### [pH]

pH of the powder-containing composition of the present disclosure is not limited in particular.

In the powder-containing composition of the present disclosure, even if the powder sediments, aggregation and solidification of the sedimented powder are suppressed. Accordingly, the powder can be redispersed easily by allowing the solvent to move.

The powder-containing composition according to the first embodiment can be manufactured by mixing each component. For example, the powder-containing composition can be manufactured by dissolving the cationic polymer in the aqueous solvent to give a cationic polymer solution, and adding the powder thereto to mix therewith. The order of adding the cationic polymer and the powder may be in other way round, or may be added simultaneously.

A powder for a aqueous solvent of the present disclosure according to a second embodiment and a method for manufacturing the same are described.

The powder for the aqueous solvent comprises a powder and a cationic polymer. The cationic polymer is considered to be adsorbed to the surface of the powder (particle). The cationic polymer is considered to be present between the particles (including secondary particles).

The powder may be same as the powder in the first embodiment described above.

The cationic polymer may be same as the cationic polymer in the first embodiment described above. Examples of the cationic polymer other than the cationic polymer shown in the first embodiment may include Polyquaternium-7, Polyquaternium-10, and the like.

The blending ratio between the powder and the cationic polymer may be same as the powder in the first embodiment described above.

According to the powder for the aqueous solvent of the second embodiment, it may be used as the powder to be added to a aqueous solvent. The applicable aqueous solvent may be same as the aqueous solvent in the first embodiment described above.

The powder for the aqueous solvent can achieve effects same as the powder-containing composition according to the first embodiment by being added to the aqueous solvent. By having it in form of a powder, the range of forms of use can be expanded and convenience can be enhanced. Moreover, the powder can be transported in a state without the aqueous solvent, and thus costs for transportation can be reduced compared to the first embodiment.

The method for manufacturing the powder for the aqueous solvent may include: a mixing step of mixing the cationic polymer and a powder in the solvent to prepare a mixture; and an isolating step of isolating the mixture from the solvent. In the mixing step, the cationic polymer may be mixed with the powder while it is dissolved in the solvent. The blending ratio between the cationic polymer and the powder in the mixing step may be same as the blending ratio between the cationic polymer and the powder in the powder-containing composition according to the first embodiment. In the mixing step, the cationic polymer and the powder may be added in any order. In the mixing step, the cationic polymer is considered to be adsorbed to the particle surface bearing a negative charge. In the isolating step, the mixture may be isolated from the solvent by decantation, filtering, evaporative removal of the solvent, and the like. In the mixture, the cationic polymer is considered to be adsorbed to the particle surface.

If the cationic polymer can be adsorbed to the particle surface of the powder, mixing of the cationic polymer and the powder may be performed not only in the liquid phase, but also in a solid phase. When mixing is performed in the solid phase, the isolating step becomes unnecessary.

### Examples

The powder-containing composition, the powder for the aqueous solvent, and the methods for manufacturing thereof of the present disclosure are described with reference to examples below. However, the composition of the present disclosure is not limited to the following examples. The contents of the components in each table are in "% by mass" unless otherwise specified.

### [Test examples 1-12]

A mixture of the powder and the cationic polymer was prepared in water to evaluate dispersibility of the mixture. Formulations and results of each component are shown in Tables 1 to 3. A non-porous talc powder that has an average particle size of 12.1 µm, has a BET specific surface area of 4.27 m²/g, is plate-shaped or lump-shaped, and has an average value of the aspect ratio of 5, and a non-porous mica powder that has an average particle size of 4.9 µm, has a BET specific surface area of 3 m²/g, is plate-shaped, and has an average value of the aspect ratio of 60 were used as the powder. Polyacrylate-1 (vinylpyrrolidone/N,N'-dimethylaminoethyl methacrylate/stearyl acrylate/tripropylene glycol diacrylate copolymer) was used as the cationic polymer. The "mass of (B)/surface area of (A)" shown in Tables 1 to 3 are a value of which the mass of the cationic polymer added is divided by the total surface area (specific surface area × added mass) of the powder.

Each composition put in a sample tube was left to stand still for three days at room temperature, and the powder sedimented. Then, the sample tube was mounted onto a table of a vibrator: MINI SHAKER MODEL M-6, manufactured by TAKASHOW, and the sample tube was shaken at SPEED 8 to evaluate redispersibility of the sedimented powder with the following criteria.
A: The sediment was completely dispersed in 10 seconds of shaking.
B: The sediment was completely dispersed in 20 seconds of shaking.
C: The sediment was completely dispersed in 30 seconds of shaking.
D: The sediment was completely dispersed in 40 seconds of shaking.
E: The sediment was not completely dispersed in 40 seconds or less of shaking.

As the addition amount of the cationic polymer relative to the total surface area of the powder was increased, improvement in dispersibility was observed. Accordingly, it is considered that the addition amount of the cationic polymer relative to the total surface area of the powder is preferably 1 µg/m² or greater, more preferably 3 µg/m² or greater, more preferably 5 µg/m² or greater, more preferably 10 µg/m² or greater, and further more preferably 15 µg/m² or greater. On the other hand, as the addition amount of the cationic polymer relative to the total surface area of the powder was further increased, deterioration in redispersibility due to generation of aggregates of the powder was observed. Accordingly, it is considered that the addition amount of the cationic polymer relative to the total surface area of the powder is preferably 10,000 µg/m² or less, more preferably 7,000 µg/m² or less, and 6,000 µg/m² or less.

Since similar tendency of dispersion was observed in talc and mica, it is considered that the mass ratio of the cationic polymer to the surface area of the powder described above is applicable to powders having the BET specific surface area and/or the particle shape that are the same as or similar to talc and/or mica.

According to Test examples 2-4, 7-10 and 12, it is considered that redispersibility can be enhanced with the powder of 10% by mass or less, at least. Moreover, in Test examples 2-4, 7-10 and 12, redispersibility could be enhanced without addition of a nonionic surfactant.

**[Table 1]**

| Test Example | 1 | 2 | 3 | 4 | 5 |
|---|---|---|---|---|---|
| (A) Talc^{*1} | 1 | 1 | 1 | 1 | 1 |
| (B) Polyacrylate-1^{*2} | - | 0.0001 | 0.001 | 0.01 | 0.1 |
| Ion-exchanged water | Balance | Balance | Balance | Balance | Balance |
| Total | 100 | 100 | 100 | 100 | 100 |
| Mass of (B)/surface ar ea of (A) (µg/m²) | 0 | 23 | 234 | 2341 | 23419 |
| Redispersibility | E | A | A | A | E |
| *1: JA-68R, ASADA MILLING CO., LTD | | | | | |
| *2: Cosquat GA468, OSAKA ORGANIC CHEMICAL INDUSTRY LTD. | | | | | |

**[Table 2]**

| Test Example | 6 | 7 | 8 | 9 | 10 |
|---|---|---|---|---|---|
| (A) Talc^{* 1} | 4 | 4 | 4 | 4 | 4 |
| (B) Polyacrylate-1^{*2} | - | 0.0001 | 0.001 | 0.01 | 0.1 |
| Ion-exchanged water | Balance | Balance | Balance | Balance | Balance |
| Total | 100 | 100 | 100 | 100 | 100 |
| Mass of (B)/surface ar ea of (A) (µg/m²) | 0 | 5.9 | 59 | 585 | 5855 |
| Redispersibility | E | C | A | A | A |

**[Table 3]**

| Test Example | 11 | 12 |
|---|---|---|
| (A) Mica^{*3} | 1 | 1 |
| (B) Polyacrylate-1^{*2} | - | 0.0001 |
| Ion-exchanged water | Balance | Balance |
| Total | 100 | 100 |
| Mass of (B)/surface area of (A) (µg/m²) | 0 | 33 |
| Redispersibility | D | C |
| *3: Y-2500, YAMAGUCHI MICA CO., LTD. | | |

### [Test examples 13-14]

The types of the cationic polymer were varied, and tests similar to the compositions according to Test examples 1-12 were performed. Formulations and results of each component are shown in Table 4. As a comparison, the composition according to Test example 4 is also shown in Table 4.

Test examples 13 and 14 that used Polyquaternium-7 and Polyquaternium-10 were low in redispersibility compared to Test example 4 in which the same amount of the cationic polymer was added. Accordingly, the polymer having the components of Polyacrylate-1 is considered to be preferable as the cationic polymer.

**[Table 4]**

| Test Example | 4 | 13 | 14 |
|---|---|---|---|
| (A) Talc^{*1} | 1 | 1 | 1 |
| (B) Polyacrylate-1^{*2} | 0.01 | - | - |
| (B) Polyquaternium-7^{*4} | - | 0.01 | - |
| (B) Polyquaternium-10^{*5} | - | - | 0.01 |
| Ion-exchanged water | Balance | Balance | Balance |
| Total | 100 | 100 | 100 |
| Mass of (B)/surface area of (A) (µg/m²) | 2341 | 2341 | 2341 |
| Redispersibility | A | C | B |
| *3: MERQUAT® 550PR, Lubrizol Advanced Materials | | | |
| *4: UCARE® Polymer JR-400, The Dow Chemical Company | | | |

### [Test examples 15 and 16]

The sedimented solid content (powder state) and the liquid in the composition prepared in Test example 4 were separated. The separated liquid was dried to measure the amount of the cationic polymer that remained in the liquid. The residue of the cationic polymer was zero g, and it became obvious that all of the cationic polymer was contained in the solid content. Accordingly, the cationic polymer is considered to be contained in the isolated powder solid content (the powder for the aqueous solvent, as used in the present disclosure).

Water was added to the isolated powder solid content to prepare the powder-containing composition again, and it was examined whether the powder solid content can be redispersed. Formulations of the prepared powder-containing compositions are shown in Table 5 (units are in grams). The amount of water in Test example 15 was decreased than in Test example 4. The amount of water in Test example 16 was increased than in Test example 4. As in Test example 4, the composition was left to stand still for three days at room temperature, and after the solid content sedimented, the composition was shaken to examine redispersibility. The test method and evaluation criteria are the same as the above-mentioned Test examples.

In any of Test examples 15 and 16, redispersibility similar to Test example 4 could be confirmed. Accordingly, the cationic polymer is considered to be adsorbed to the surface of each particle of the powder in the powder-containing composition. Moreover, in the powder for the aqueous solvent as used in the present disclosure, it was confirmed that aggregation and solidification are suppressed in the aqueous solvent, and the powder can be easily redispersed.

Moreover, in Test examples 4, 15 and 16 in which the amounts of the solvent were varied, no difference in redispersibility was observed. Accordingly, in the powder-containing composition of the present disclosure, it is considered that dispersibility of the powder does not depend on the amount of the aqueous solvent, but depends on the blending ratio between the cationic polymer and the powder.

**[Table 5]**

| Test Example | 15 | 16 |
|---|---|---|
| Solid content isolated from the powder-containi ng composition of Test example 4 | 1.001g | 1.001g |
| Ion-exchanged water | Balance | Balance |
| Total | 50g | 110g |
| Mass of (B)/surface area of (A) (µg/m²) | 2341 | 2341 |
| Redispersibility | A | A |

The formulation examples of the powder-containing composition of the present disclosure are listed below. Examples of application of the powder-containing composition of the present disclosure are not limited to the formulation examples listed below.

### Formulation example 1: Powder-containing cosmetic lotion (Table 6)

**[Table 6]**

| | Component | Content (mass%) |
|---|---|---|
| (1) | (A) Talc^{*1} | 1 |
| (2) | (B) Polyacrylate-1^{*2} | 0.01 |
| (3) | Water | 73.24 |
| (4) | Ethanol | 15 |
| (5) | Glycerin | 1.5 |
| (6) | Dipropylene glycol | 2 |
| (7) | Xylitol | 1 |
| (8) | PEG-20 | 3 |
| (9) | PEG/PPG-14/Dimethyl ether | 1 |
| (10) | P PEG/PPG-17/Dimethyl ether | 1 |
| (11) | PPG-13 Decyltetradeceth-24 | 0.1 |
| (12) | Sodium chloride | 0.2 |
| (13) | Succinic acid | 0.15 |
| (14) | Disodium succinate | 0.35 |
| (15) | Phenoxyethanol | 0.3 |
| (16) | EDTA-3Na | 0.1 |
| (17) | Perfume | 0.05 |
| Total | | 100 |
| Mass of (B)/surface area of (A) (µg/m²) | | 2341 |

### Formulation example 2: Skin external agent for body sheets (Table 7)

**[Table 7]**

| | Component | Content (mass%) |
|---|---|---|
| (1) | (A) Talc^{*1} | 1 |
| (2) | (B) Polyacrylate-1^{*2} | 0.01 |
| (3) | Water | 42.22 |
| (4) | Lactic acid | 0.03 |
| (5) | Sodium lactate | 0.17 |
| (6) | EDTA-3Na | 0.01 |
| (7) | Glycerin | 2 |
| (8) | Dipropylene glycol | 8 |
| (9) | PPG-13 Decyltetradeceth-24 | 0.06 |
| (10) | Ethanol | 45 |
| (11) | Perfume | 1.5 |
| Total | | 100 |
| Mass of (B)/surface area of (A) (µg/m²) | | 2341 |

### Formulation example 3: After makeup lotion (Table 8)

**[Table 8]**

| | Component | Content (mass%) |
|---|---|---|
| (1) | (A) Talc^{*1} | 1 |
| (2) | (B) Polyacrylate-1^{*2} | 0.01 |
| (3) | Water | 73.405 |
| (4) | Lactic acid | 0.005 |
| (5) | Sodium lactate | 0.5 |
| (6) | Ethanol | 20 |
| (7) | PPG-13 Decyltetradeceth-24 | 0.03 |
| (8) | Bis-PEG-18 methyl ether dimethyl silane | 2 |
| (9) | Crystalline cellulose | 1 |
| (10) | Polyurethane-10 | 1 |
| (11) | PPG-12 Dimethicone | 1 |
| (12) | Perfume | 0.05 |
| Total | | 100 |
| Mass of (B)/surface area of (A) (µg/m²) | | 2341 |

### Formulation example 4: Powder-containing hair product (Table 9)

**[Table 9]**

| | Component | Content (mass%) |
|---|---|---|
| (1) | (A) Talc^{*1} | 1 |
| (2) | (B) Polyacrylate-1^{*2} | 0.01 |
| (3) | Water | 43.69 |
| (4) | Ethanol | 50 |
| (5) | Sodium lauryl sulfate | 0.2 |
| (6) | PPG-13 Decyltetradeceth-24 | 2 |
| (7) | Sodium Methyl Stearoyl Taurate | 0.05 |
| (8) | PEG-6 | 1 |
| (9) | PEG-32 | 1 |
| (10) | Glycerin | 1 |
| (11) | Perfume | 0.05 |
| Total | | 100 |
| Mass of (B)/surface area of (A) (µg/m²) | | 2341 |

### Industrial applicability

The powder-containing composition, the powder for the aqueous solvent, and the methods for manufacturing thereof of the present disclosure have been described according to the foregoing embodiments and examples, but they are not limited to the foregoing embodiments and examples and may encompass various transformations, modifications, and improvements made to the various disclosed elements (including elements disclosed in the Claims, Description, and Drawings) within the scope of the invention and according to the fundamental technical idea of the invention. Further, various combinations, substitutions, and selections of the various disclosed elements are possible within the scope of the claims of the invention.

Further issues, objectives, and embodiments (including modifications) of the invention are revealed also from the entire disclosure of the invention including the Claims.

The numerical ranges disclosed herein are to be construed in such a manner that arbitrary numerical values and ranges falling within the disclosed ranges are treated as being concretely described herein, even where not specifically stated.

The powder-containing composition, the powder for the aqueous solvent, and the methods for manufacturing thereof of the present disclosure can be applied generally to products that use powders in aqueous solvents. For example, the powder-containing composition, the powder for the aqueous solvent, and the methods for manufacturing thereof of the present disclosure can be applied to cosmetics, cleansing agents, paints, inks, foods and the like.

## Claims

1. A powder-containing composition comprising:
an aqueous solvent;
a cationic polymer that is present in said aqueous solvent; and
a powder that bears a negative charge on a surface in said aqueous solvent and has a BET specific surface area of less than 50 m²/g;
wherein a ratio of a mass in the composition of said cationic polymer to a total surface area in the composition of said powder is 1 µg/m² to 100,000 µg/m².

2. The composition according to claim 1, wherein said cationic polymer comprises, relative to a molecular weight of said cationic polymer:
15% to 85% of a first component having a structure represented by Chem. 1; and
15% to 85% of a second component having a structure represented by Chem. 2 or Chem. 3. 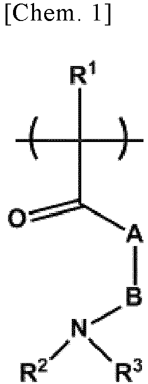 (in Chem. 1: R¹ represents a hydrogen atom or a methyl group; R² and R³ each independently represent a hydrogen atom, a methyl group, an ethyl group, or a t-butyl group; A represents an oxygen atom or an NH group; and B represents a C₁₋₄ alkylene group that is linear or has a side chain.) 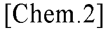 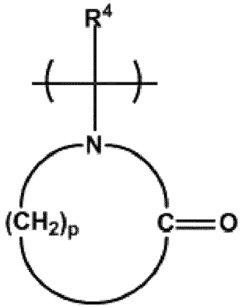 (in Chem. 2: R⁴ represents a hydrogen atom or a methyl group; and p represents 3 or 4.) 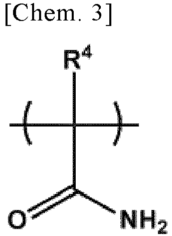 (in Chem. 3: R⁴ represents a hydrogen atom or a methyl group.)

3. The composition according to claim 1 or 2, wherein said cationic polymer is adsorbed to said powder.

4. The composition according to any one of claims 1 to 3, wherein said powder has an aspect ratio of two or more, the aspect ratio being an average value of a ratio of a second length in a longitudinal direction to a first length in a transverse direction in each particle.

5. The composition according to any one of claims 1 to 4, wherein said powder has a BET specific surface area of 1 m²/g to 30 m²/g.

6. The composition according to any one of claims 1 to 5, wherein said powder is at least one of talc and/or mica.

7. The composition according to any one of claims 1 to 6, wherein a content of said powder relative to the mass of the composition is 0.2% by mass to 10% by mass.

8. The composition according to any one of claims 1 to 7, wherein a content of said cationic polymer relative to the mass of the composition is 0.00005% by mass to 0.5% by mass.

9. The composition according to any one of claims 1 to 8, wherein said cationic polymer further comprises, relative to the molecular weight of said cationic polymer:
0.1% to 10% of a third component having a structure represented by Chem. 4. 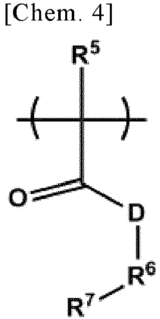 (in Chem. 4: R⁵ represents a hydrogen atom or a methyl group; D represents an oxygen atom or an NH group; R⁶ represents a C1-17 alkylene group that is linear or has a side chain, or a group represented by Chem. 5; and R⁷ represents a hydrogen atom or a methyl group.) 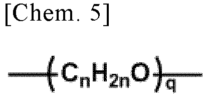 (in Chem. 5: n represents an integer of 1 to 4; and q represents an integer of 1 to 25.)

10. The composition according to any one of claims 1 to 9, wherein said cationic polymer further comprises, relative to the molecular weight of said cationic polymer:
0.1% to 15% of a fourth component having a structure represented by Chem. 6. 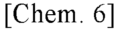 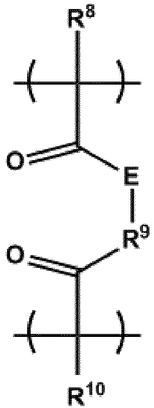 (in Chem. 6: R⁸ represents a hydrogen atom or a methyl group; E represents an oxygen atom or an NH group; R⁹ represents a C₁₋₁₇ alkylene group that is linear or has a side chain, or a group represented by Chem. 7; and R¹⁰ represents a hydrogen atom or a methyl group.) 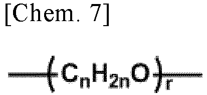 (in Chem. 7: n represents an integer of 1 to 4, and r represents an integer of 1 to 25.)

11. The composition according to any one of claims 1 to 10, wherein said cationic polymer is vinylpyrrolidone/N,N'-dimethylaminoethyl methacrylate/stearyl acrylate/tripropylene glycol diacrylate copolymer.

12. The composition according to any one of claims 1 to 11, wherein a content of a nonionic surfactant relative to the mass of the composition is 0.1% by mass or less.

13. The composition according to any one of claims 1 to 12, wherein a content of an oil component relative to the mass of the composition is 5% by mass or less.

14. A powder for an aqueous solvent comprising:
a particle bearing a negative charge on a surface in the aqueous solvent and having a BET specific surface area of less than 50 m²/g; and
a cationic polymer adhered to the surface of said particle,
wherein a ratio of a mass of said cationic polymer to a total surface area of said particle is 1 µg/m² to 100,000 µg/m².

15. The powder according to claim 14, wherein said cationic polymer comprises, relative to a molecular weight of said cationic polymer:
15% to 85% of a first component having a structure represented by Chem. 8; and
15% to 85% of a second component having a structure represented by Chem. 9 or Chem. 10, 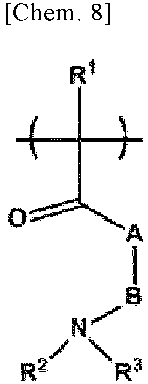 (in Chem. 8: R¹ represents a hydrogen atom or a methyl group; R² and R³ each independently represent a hydrogen atom, a methyl group, an ethyl group, or a t-butyl group; A represents an oxygen atom or an NH group; and B represents a C₁₋₄ alkylene group that is linear or has a side chain.) 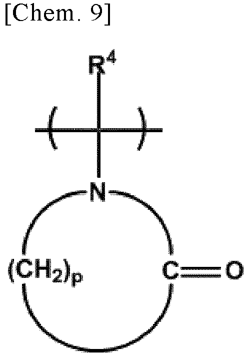 (in Chem. 9: R⁴ represents a hydrogen atom or a methyl group; and p represents 3 or 4.) 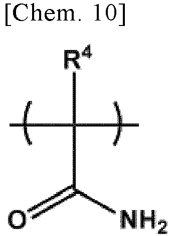 (in Chem. 10: R⁴ represents a hydrogen atom or a methyl group.)

16. The powder according to claim 14 or 15, wherein said particle has an aspect ratio of two or greater, the aspect ratio being an average value of a second length in a longitudinal direction to a first length in a transverse direction.

17. The powder according to any one of claims 14 to 16, wherein said particle has a BET specific surface area of 1 m²/g to 30 m²/g.

18. The powder according to any one of claims 14 to 17, wherein said particle is at least one of talc and/or mica.

19. The powder according to any one of claims 14 to 18, wherein said cationic polymer further comprises, relative to a molecular weight of said cationic polymer:
0.1% to 10% of a third component having a structure represented by Chem. 11. 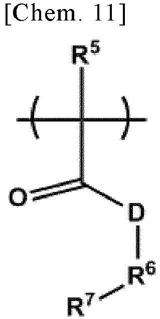 (in Chem. 11: R⁵ represents a hydrogen atom or a methyl group; D represents an oxygen atom or an NH group; R⁶ represents a C₁₋₁₇ alkylene group that is linear or has a side chain, or a group represented by Chem. 5; and R⁷ represents a hydrogen atom or a methyl group.) 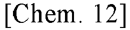 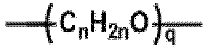 (in Chem. 12: n represents an integer of 1 to 4; and q represents an integer of 1 to 25.)

20. The powder according to any one of claims 14 to 19, wherein said cationic polymer further comprises, relative to the molecular weight of said cationic polymer:
0.1% to 15% of a fourth component having a structure represented by Chem. 13. 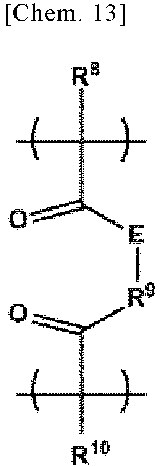 (in Chem. 13: R⁸ represents a hydrogen atom or a methyl group; E represents an oxygen atom or an NH group; R⁹ represents a C₁₋₁₇ alkylene group that is linear or has a side chain, or a group represented by Chem. 14; and R¹⁰ represents a hydrogen atom or a methyl group.) 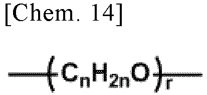 (in Chem. 14: n represents an integer of 1 to 4; and r represents an integer of 1 to 25.)

21. The powder according to any one of claims 14 to 20, wherein said cationic polymer is vinylpyrrolidone/N,N'-dimethylaminoethyl methacrylate/stearyl acrylate/tripropylene glycol diacrylate copolymer.

22. A method for manufacturing a powder for an aqueous solvent comprising:
mixing, in the aqueous solvent, a powder that bears a negative charge on a surface in an aqueous solvent and has a BET specific surface area of less than 50 m²/g, and 1 µg/m² to 100,000 µg/m² of a cationic polymer relative to a total surface area of said powder; and
isolating the mixture prepared in the mixing step from said aqueous solvent.
